# FASCICULE DE BREVET EUROPEEN

(11) **EP 0 509 922 B1**
(45) Date de publication et mention de la délivrance du brevet: **26.06.1996**
(21) Numéro de dépôt: 92401061.4
(22) Date de dépôt: 15.04.1992
(51) Int. Cl.: A61K 7/06

(54) **Composition et procédé mettant en oeuvre des silicones thiols pour la protection de la couleur de fibres kératiniques teintes**
Mittel und Verfahren unter Verwendung Silikonthiolen zum Schutz der Farbe bei keratinisch gefärbten Fasern
Composition and method using silicones thiols for the colour protection of dyed keratinic fibres

(30) Priorité: 19.04.1991 FR 9104886
(43) Date de publication de la demande: 21.10.1992
(73) Titulaire: L'OREAL, F-75008 Paris (FR)
(72) Inventeur: Dubief, Claude, F-78150 Le Chesnay (FR); Cauwet, Danièle, F-75011 Paris (FR); Millequant, Jean-Marie, F-94100 Saint-Maur (FR)
(74) Mandataire: Casalonga, Axel

(56) Documents cités:
- EP-A- 0 295 780
- EP-A- 0 351 297
- FR-A- 1 356 552
- FR-A- 1 378 791
- GB-A- 1 182 939
- GB-A- 1 199 776

## Description

La présente invention a pour objet l'utilisation de silicones à groupements thiols en tant qu'agents de protection de la coloration de cheveux teints, aux compositions et aux procédés mettant en oeuvre ces silicones à groupements thiols.

On sait depuis longtemps que notamment la lumière et les agents de lavage ont tendance à agresser la couleur artificielle des cheveux teints, cette couleur s'affaiblissant peu à peu ou virant vers des couleurs peu esthétiques ou non souhaitables.

Il est donc apparu nécessaire de protéger la couleur des fibres kératiniques teintes et plus particulièrement des cheveux teints, vis-à-vis des agents extérieurs susceptibles d'affaiblir ou de faire virer la coloration initiale obtenue après teinture.

Le document GB-A-1199776 décrit des compositions cosmétiques comprenant des copolymères d'organosilicones incluant des motifs 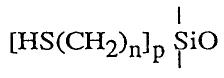
où n est un entier au moins égal à 2 et p est égal à 0, 1 ou 2. Ces copolymères sont utiles pour le traitement des cheveux, en particulier pour obtenir un agencement permanent des cheveux en présence d'une humidité importante sans affecter la couleur naturelle des cheveux.

Le document EP-A-0295780 décrit un procédé de traitement des cheveux qui comprend l'application d'une composition contenant un agent réducteur des liaisons disulfure, tel qu'un thioglycolate d'ammonium et un composé d'organosilicone ayant au moins un atome de silicium lié à un groupe mercaptoalkyle. Les cheveux sont alors arrangés dans la configuration voulue et les liaisons disulfure des cheveux sont ensuite restaurées par application d'un agent oxydant.

Le document GB 1182939 décrit un procédé de traitement des cheveux qui comprend la réduction des liaisons disulfure des cheveux par application d'un agent réducteur, l'arrangement des cheveux dans la configuration voulue, la réoxydation des liaisons disulfure réduites et l'application d'une dispersion aqueuse d'un composé d'organosilicones contenant au moins un substituant mercaptoalkyle après la réduction et avant la réoxydation des liaisons disulfure. Ce procédé a pour objet de rendre plus attractif l'arrangement des cheveux pendant des périodes de temps plus longues tout en conservant la couleur naturelle des cheveux.

Enfin, le document FR-A-1356552 décrit un procédé de préparation de silicones thiols mais n'envisage aucune application particulière de ces silicones thiols.

La demanderesse a découvert, ce qui fait l'objet de l'invention, que des silicones portant des groupements thiols préservaient la coloration artificielle des fibres kératiniques teintes et en particulier des cheveux, des dégradations dues notamment à la lumière et au lavage. Cette propriété a été mise en évidence en particulier par l'exposition à la lumière artificielle d'un simulateur solaire du type XENOTEST 150 d'ORIGINAL HANAU ou par lavage dans des conditions standards à l'aide d'une machine AHIBA TEXOMAT G6B.

L'invention a donc pour objet l'utilisation de silicones thiols en tant qu'agents de protection de la couleur des fibres kératiniques teintes, en particulier des cheveux teints.

Un autre objet de l'invention est constitué par de nouvelles compositions comprenant des silicones thiols utiles comme agents de protection de la couleur des fibres kératiniques teintes, en particulier des cheveux teints.

D'autres objets de l'invention apparaitront à la lecture de la description et des exemples qui suivent.

La présente invention concerne l'utilisation comme agent de protection de la couleur des fibres kératiniques teintes d'un polydiorganosiloxane à fonction thiol comportant dans sa molécule au moins une unité de formule : 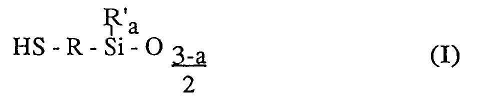
dans laquelle R désigne un radical aliphatique divalent ayant de 3 à 26 atomes de carbone, éventuellement interrompu par une fonction ester et pouvant porter des motifs d'oxyde d'éthylène ou d'oxyde de propylène ou leur mélange, R' désigne un radical monovalent hydrocarbonné ayant 1 à 6 atomes de carbone, alcoxy ayant 1 à 4 atomes de carbone, triméthylsilyloxy ; a désigne un nombre entier égal à 0, 1 ou 2,
les unités restantes ayant pour formule :
1) 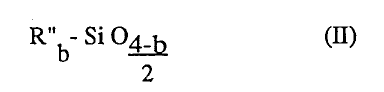 dans laquelle :
   R'' représente un groupe alkyle en C₁-C₁₈, phénylalkyle(C₁-C₆) ou phényle;
   b est un nombre entier désignant 1, 2, ou 3;
   au moins 50% des groupements R' et R'' représentant un groupement méthyle, et
2) éventuellement des unités de formule : 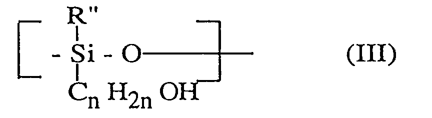 La présente invention concerne également de nouvelles compositions destinées à être appliquées sur des fibres kératiniques teintes et en particulier les cheveux humains teints pour les protéger de la dégradation due à des agents extérieurs contenant dans un milieu approprié pour l'application sur les fibres kératiniques au moins 0,1 % d'un polydiorganosiloxane à fonction thiol comportant dans sa molécule au moins une unité de formule : 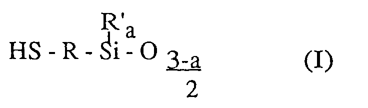 dans laquelle :
   R désigne un groupement de formule : 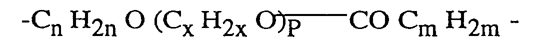 dans laquelle n désigne un nombre entier compris entre 1 et 18, m désigne un nombre entier compris entre 1 et 8, x = 2 ou 3 et lorsque x = 3 le radical C₃H₆ est ramifié, p est égal à 0 ou désigne un nombre pouvant aller jusqu'à 40;
   R' désigne un radical monovalent hydrocarboné ayant 1 à 6 atomes de carbone, alcoxy ayant 1 à 4 atomes de carbone, triméthylsilyloxy;
   a désigne un nombre entier égal à 0, 1 ou 2, les unités restantes ayant pour formule : 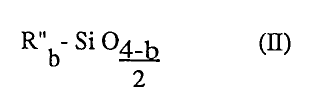 dans laquelle :
   R'' représente un groupement alkyle en C₁-C₁₈, phénylalkyle (C₁-C₆) ou phényle;
   b est un nombre entier désignant 1, 2, ou 3; au moins 50 % des groupements R' et R'' représentant un radical méthyle.

Le polydiorganosiloxane à fonction thiol de ces nouvelles compositions peut également comporter des unités de formule (III).

Le taux pondéral des groupements thiols présents dans le polydiorganosiloxane utilisés conformément à l'invention est compris entre 0,1 et 15 % et de préférence entre 0,15 et 13 %.

Le radical R peut représenter notamment un groupement alkylène ayant 3 à 8 atomes de carbone tel que plus particulièrement un groupement (CH₂)ₙ où n est compris entre 3 et 8, ou un groupement de formule : 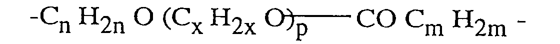
dans laquelle n désigne un nombre entier compris entre 1 et 18, m désigne un nombre entier compris entre 1 et 8, x = 2 ou 3 et lorsque x = 3 le radical C₃H₆ est ramifié p est égal à 0 ou désigne un nombre pouvant aller jusqu'à 40.

R' désigne plus particulièrement un radical alkyle en C₁-C₆ tel que méthyle, éthyle, n-propyle, isopropyle, n-butyle.

De préférence, le radical R'' représente un groupement méthyle, C₁₂H₂₅ , 2-phénylpropyle;
le radical R représente -(CH₂)₃- , 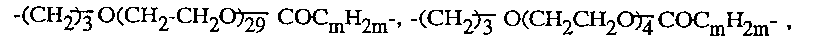 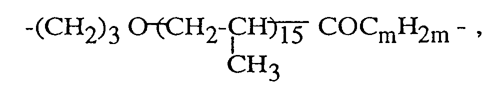
-(CH₂)₃O CO CH₂- , 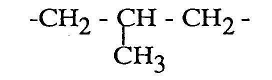
-(CH₂)₁₂-
et le radical R' représente méthyle ou triméthylsilyloxy.

Le nombre total des unités (I), (II) et éventuellement (III) est de préférence égal ou inférieur à 500 et compris en particulier entre 10 et 500.

La demanderesse a constaté que les polydiorganosiloxanes à fonction thiol définis ci-dessus étaient particulièrement efficaces pour préserver la couleur de cheveux teints des agressions de la lumière et des lavages.

Les polydiorganosiloxanes à fonction thiol définis ci-dessus sont utilisés de préférence dans des quantités au moins égales à 0,1 % et généralement comprises entre 0,1 et 20 % et de préférence entre 1 et 10 % dans une composition contenant un milieu cosmétiquement acceptable, approprié pour être appliqué sur les fibres kératiniques, en particulier les cheveux humains.

De telles compositions destinées à être appliquées sur les fibres kératiniques teintes et en particulier sur les cheveux teints en vue de protéger leur couleur artificielle, se présentent sous forme de lotions huileuses ou alcooliques, d'émulsions, de dispersions aqueuses ou hydroalcooliques.

Lorsque les compositions destinées à être appliquées sur les fibres kératiniques teintes et en particulier les cheveux constituent des lotions huileuses, elles contiennent outre le polydiorganosiloxane à fonction thiol, des huiles minérales, végétales, animales ou synthétiques et plus particulièrement des isoparaffines telles que les ISOPARS ou des huiles de silicone telles que des huiles de silicone à structure linéaire ou cyclique comme les polyalkylsiloxanes, les polyarylsiloxanes, les polyalkylarylsiloxanes ou les polyorganosiloxanes modifiés par des groupements organofonctionnels différents des groupements thiols définis ci-dessus ou leur(s) mélange(s).

Les lotions huileuses peuvent également contenir des cires, résines ou gommes de silicone conjointement aux huiles mentionnées ci-dessus.

Les lotions alcooliques contiennent, outre le polydiorganosiloxane à fonction thiol, un alcool inférieur comportant 2 à 4 atomes de carbone et de préférence l'éthanol ou l'isopropanol et éventuellement d'autres solvants organiques comme les alkylèneglycols ou les éthers de glycols.

Lorsque les compositions pour fibres kératiniques teintes conformes à l'invention se présentent sous forme d'émulsions, elles constituent des émulsions non-ioniques ou cationiques et de préférence non-ioniques.

La phase grasse des émulsions est constituée, soit uniquement par le polydiorganosiloxane à fonction thiol tel que défini ci-dessus, soit par un mélange de ce polydiorganosiloxane avec d'autres huiles ou cires telles que celles mentionnées ci-dessus pour les lotions huileuses.

L'autre phase des émulsions est constituée par un milieu aqueux.

Les émulsions non-ioniques contiennent un émulsionnant nonionique choisi parmi les alcools gras polyoxyéthylénés, les acides gras polyoxyéthylénés, les esters du sorbitan éventuellement polyoxy éthylénés, les alkylphénols polyoxyéthylénés ou polyglycérolés, les amides gras polyoxyéthylénés ou polyglycérolés, les alcools gras et alpha-diols polyglycérolés ; le nombre de groupements polyoxyéthylénés étant compris entre 2 et 50, le nombre de groupements polyglycéroles étant compris entre 2 et 30 ; les amides gras polyoxyéthylénés comportent de préférence 2 à 30 moles d'oxyde d'éthylène; les amides gras polyglycérolés comportent de préférence 1 à 5 groupements glycérol et en particulier 1,5 à 4; les esters d'acides gras du sorbitan comportent de préférence 2 à 30 moles d'oxyde d'éthylène.

Les émulsions cationiques contiennent un émulsionnant cationique choisi parmi les halogénures d'ammonium quaternaire tels que de dialkyl (C₁₀-C₃₀) diméthylammonium, d'alkyl (C₁₀-C₃₀) triméthyl ammonium ou d'alkyl (C₁₀-C₃₀) benzyl diméthylammonium et les sels d'ammonium quaternaires polyoxyéthylénés comportant de 2 à 30 moles d'oxyde d'éthylène. On utilise de préférence le chlorure de distéaryldiméthyl ammonium et le chlorure de béhényl triméthylammonium.

Lorsque les compositions destinées à être appliquées sur les fibres kératiniques teintes et contenant le polydiorganosiloxane à fonction thiol, utilisées conformément à l'invention sont des dispersions, elles contiennent en plus de cet agent, de l'eau et un agent de dispersion ou un agent de mise en suspension de la silicone dans le milieu aqueux. Des agents de dispersion plus particulièrement préférés sont choisis parmi un copolymère d'acrylate d'ammonium et d'acrylamide réticulé, un polyacrylamide, un polymère d'acide acrylique réticulé

Ces dispersions peuvent constituer des produits non rincés ou rincés et également des shampooings. Dans ce dernier cas, elles contiennent des tensio-actifs anioniques, non ioniques, amphotères ou leurs mélanges dans des concentrations généralement comprises entre 5 et 30% en poids.

Lorsque les compositions conformes à l'invention contiennent des solvants, ils sont choisis plus particulièrement parmi les alcanols inférieurs tels que l'éthanol.

Les compositions conformes à l'invention peuvent également renfermer tout autre agent habituellement appliqué sur les fibres kératiniques ; lorsqu'il s'agit de cheveux, ces adjuvants sont des adjuvants cosmétiquement acceptables. Ces adjuvants peuvent être choisis en particulier parmi des colorants, des conservateurs, des résines cosmétiques, des agents adoucissants, des parfums, etc.

Les compositions conformes à l'invention peuvent également se présenter sous forme de sprays ou être préssurisées dans des dispositifs aérosols.

Le procédé de protection de la couleur artificielle des fibres kératiniques teintes et en particulier de cheveux teints conforme à l'invention consiste à appliquer sur celles-ci une composition contenant au moins 0,1% d' un polydiorganosiloxane à fonction thiol comportant les unités de formules (I) et (II) telles que définies ci-dessus.

Les fibres kératiniques et en particulier les cheveux peuvent être teints en mettant en oeuvre des colorants classiquement utilisés en teinture capillaire tels que par l'utilisation de précurseurs de colorants d'oxydation suivant un processus de coloration mettant en oeuvre un agent oxydant ou par coloration dite directe mettant en oeuvre des colorants nitrés benzéniques, des colorants anthraquinoniques, azoïques classiquement utilisés en teinture des fibres kératiniques.

L'utilisation des silicones thiols selon l'invention est particulièrement bien adaptée aux cheveux teints par un processus de coloration mettant en oeuvre un agent oxydant.

De telles compositions sont bien connues du technicien et sont décrites, par exemple, dans l'ouvrage HARRY'S COSMETICOLOGY, 7ème édition, 1982, pages 533 à 545.

Les exemples suivants sont destinés à illustrer l'invention sans pour autant présenter un caractère limitatif.

### EXEMPLE 1

On prépare la composition suivante :

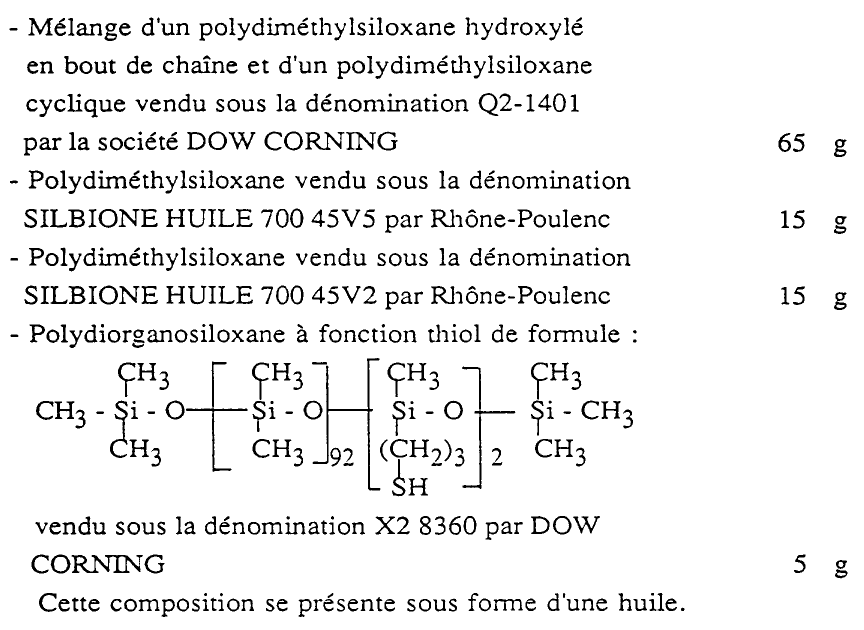

### EXEMPLE 2

On prépare la composition suivante :

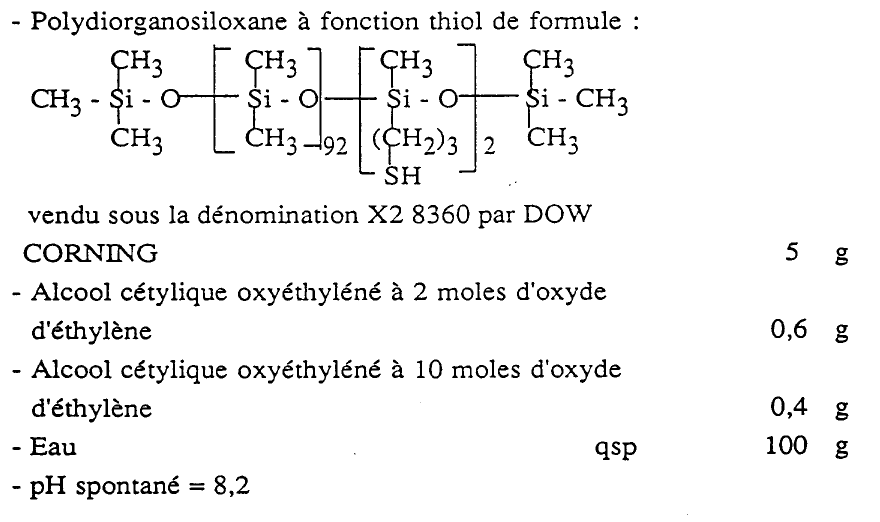

### EXEMPLE 3

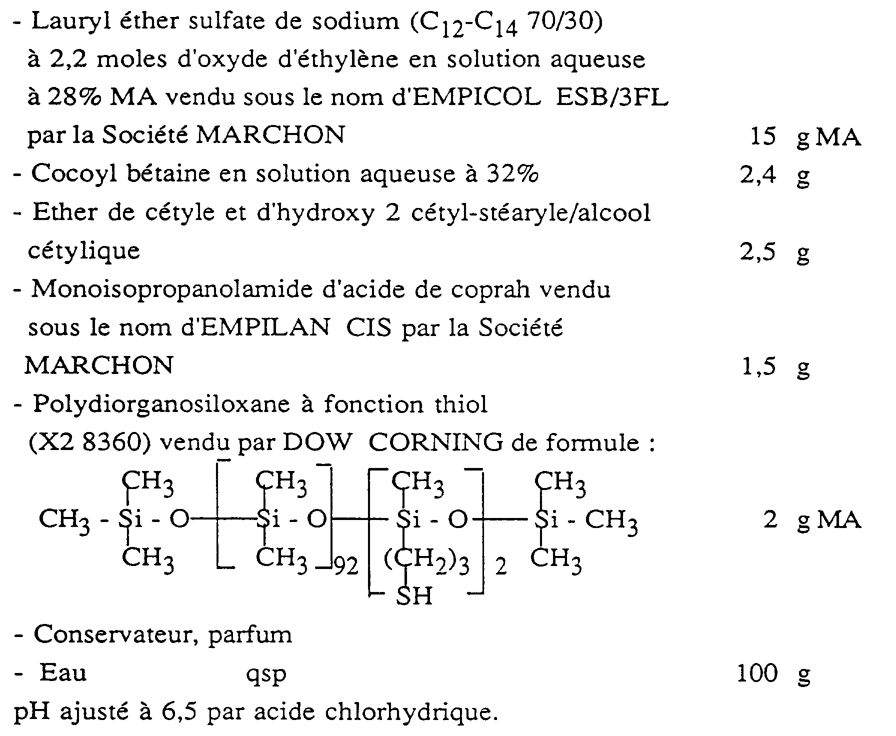

Cette composition est utilisée comme shampooing pour le lavage des cheveux.

### EXEMPLE 4

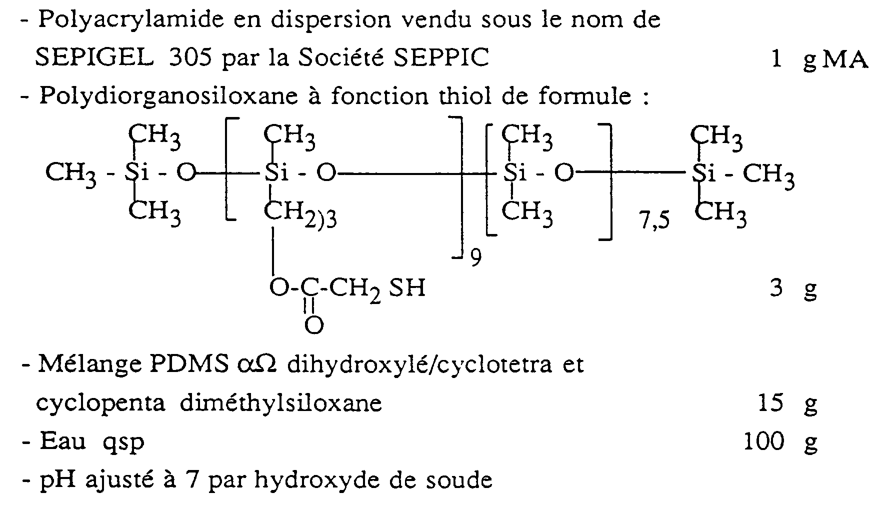

Cette composition est utilisée en application non suivie d'un rinçage pour le soin des cheveux teintés.

### EXEMPLE 5

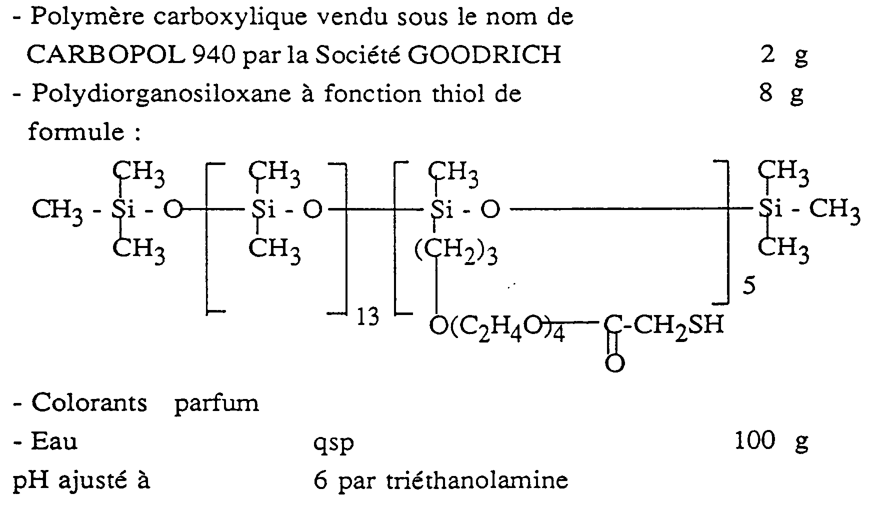

Cette composition est appliquée sur les cheveux teints, qui sont ensuite séché et coiffé sans rinçage intermédiaire.

### EXEMPLE 6

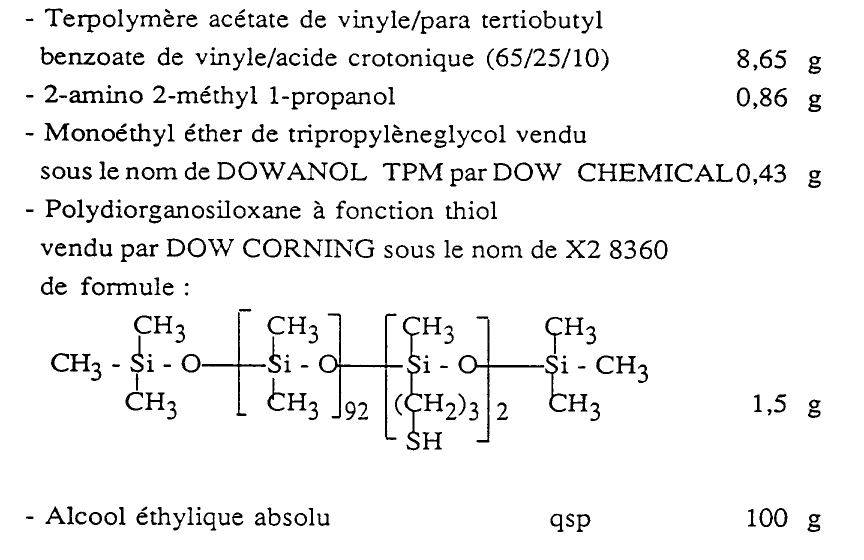

Cette composition est utilisée comme laque pour fixer les cheveux.

### EXEMPLE 7

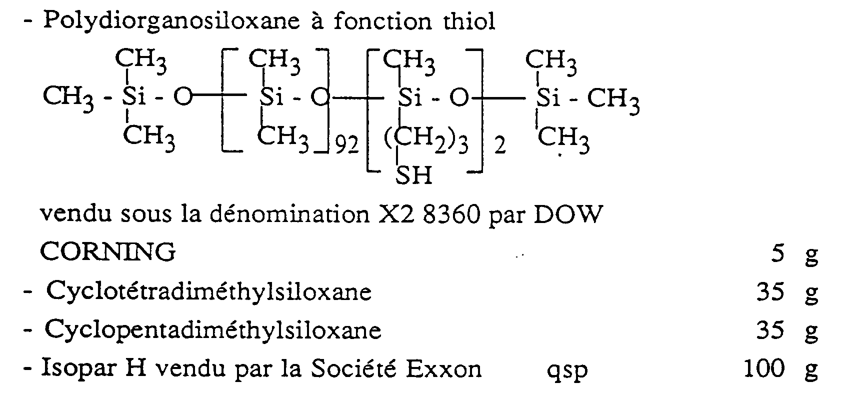

Les compositions des exemples 1 et 2 appliquées régulièrement sur des cheveux teints protègent leur couleur vis-à-vis des lavages et de la lumière tout en leur conférant de la douceur, du brillant et de la légèreté.

## Revendications

1. Utilisation d'un polydiorganosiloxane à fonction thiol comportant dans sa molécule au moins une unité de formule : 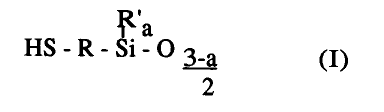 dans laquelle :
R désigne un radical aliphatique divalent ayant de 3 à 26 atomes de carbone éventuellement interrompu par une fonction ester et pouvant porter des motifs d'oxyde d'éthylène ou d'oxyde de propylène ou leurs mélanges;
R' désigne un radical monovalent hydrocarboné ayant 1 à 6 atomes de carbone, alcoxy ayant 1 à 4 atomes de carbone, triméthylsilyloxy ;
a désigne un nombre entier égal à 0, 1 ou 2, les unités restantes ayant pour formule : 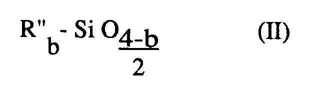 dans laquelle :
R'' représente un groupement alkyle en C₁-C₁₈, phénylalkyle (C₁-C₆) ou phényle;
b est un nombre entier désignant 1, 2, ou 3; au moins 50 % des groupements R' et R'' représentant un radical méthyle, comme agent de protection de la couleur de fibres kératiniques teintes.

2. Utilisation selon la revendication 1 dans laquelle le polyorganosiloxane comporte en plus des unités de formule : 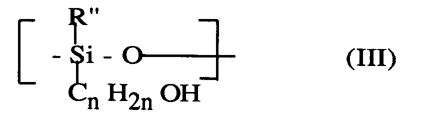 R'' a la signification indiquée dans la revendication 1 et n est un nombre entier de 1 à 18.

3. Utilisation selon la revendication 1 ou 2 dans laquelle le polyorganosiloxane comprend une unité de formule (I) dans laquelle R représente un groupement alkylène ayant 3 à 8 atomes de carbone, ou un groupement de formule : 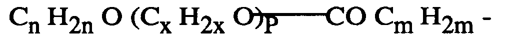 dans laquelle n désigne un nombre entier compris entre 1 et 18, m désigne un nombre entier compris entre 1 et 8, x = 2 ou 3 et lorsque x = 3 le radical C₃H₆ est ramifié, p est égal à 0 ou désigne un nombre pouvant aller jusqu'à 40.

4. Utilisation selon l'une quelconque des revendications 1 à 3, caractérisée par le fait que le polydiorganosiloxane à fonction thiol répondant à la définition indiquée dans la revendication 1 est choisi parmi les composés dans lesquels R représente :
-(CH₂)₃-, 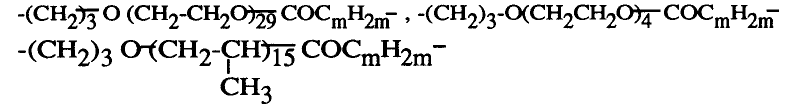 -(CH₂)₃O-COCH₂- 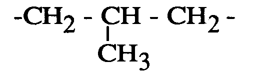 R' représente CH₃ ou triméthylsilyloxy, R'' représente CH₃, C₁₂H₂₅, 2-phénylpropyle, le nombre total des unités (I) et (II) étant inférieur ou égal à 500.

5. Utilisation selon l'une quelconque des revendications 1 à 4, caractérisée par le fait que l'on met en oeuvre des polydiorganosiloxanes à fonction thiol dans lesquels le taux pondéral de groupements thiols présents est compris entre 0,1 et 15 % et de préférence entre 0,15 et 13 %.

6. Utilisation selon l'une quelconque des revendications 1 à 5, caractérisée par le fait que le polydiorganosiloxane à fonction thiol est utilisé en mettant en oeuvre une composition contenant au moins 0,1 % en poids dudit polydiorganosiloxane à fonction thiol dans un support approprié pour être appliqué sur les libres kératiniques teintes.

7. Composition destinée à être appliquée sur des fibres kératiniques teintes et en particulier les cheveux humains teints pour les protéger de la dégradation due à des agents extérieurs, caractérisée par le fait qu'elle contient dans un milieu approprié pour l'application sur les fibres kératiniques au moins 0,1 % d'un polydiorganosiloxane à fonction thiol comportant dans sa molécule au moins une unité de formule : 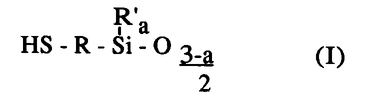 dans laquelle :
R désigne un groupement de formule : 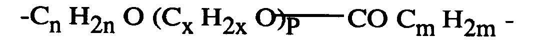 dans laquelle n désigne un nombre entier compris entre 1 et 18, m désigne un nombre entier compris entre 1 et 8, x = 2 ou 3 et lorsque x = 3 le radical C₃H₆ est ramifié, p est égal à 0 ou désigne un nombre pouvant aller jusqu'à 40;
R' désigne un radical monovalent hydrocarboné ayant 1 à 6 atomes de carbone, alcoxy ayant 1 à 4 atomes de carbone, triméthylsilyloxy ;
a désigne un nombre entier égal à 0, 1 ou 2, les unités restantes ayant pour formule : 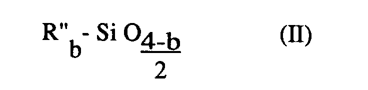 dans laquelle :
R'' représente un groupement alkyle en C₁-C₁₈, phénylalkyle (C₁-C₆) ou phényle;
b est un nombre entier désignant 1, 2, ou 3; au moins 50 % des groupements R' et R'' représentant un radical méthyle.

8. Composition selon la revendication 7 dans laquelle le polyorganosiloxane comporte en plus des unités de formule : 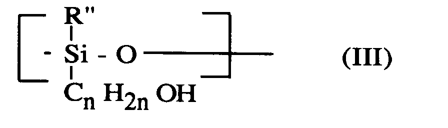 R'' a la signification indiquée dans la revendication 7 et n est un nombre entier de 1 à 18.

9. Composition selon la revendication 7 ou 8 caractérisée par le fait que le polydiorganosiloxane à fonction thiol est choisi parmi les composés dans lesquels R représente : 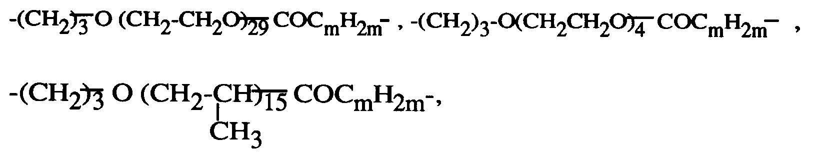 et -(CH₂)₃O-COCH₂-R' représente CH₃ ou triméthylsilyloxy, R'' représente CH₃, C₁₂H₂₅, 2-phénylpropyle, le nombre total des unités (I) et (II) étant inférieur ou égal à 500.

10. Composition selon l'une quelconque des revendications 7 à 9 caractérisée par le fait que l'on met en oeuvre des polydiorganosiloxanes à fonction thiol dans lesquels le taux pondéral de groupements thiols présents est compris entre 0,1 et 15 % et de préférence entre 0,15 et 13 %.

11. Composition selon l'une quelconque des revendications 7 à 10, caractérisée par le fait que la composition se présente sous forme de lotion huileuse, d'émulsion, de dispersion aqueuse ou hydroalcoolique.

12. Composition selon la revendication 11, caractérisée par le fait que les lotions huileuses contiennent outre le polydiorganosiloxane à fonction thiol, des huiles minérales, végétales, animales ou synthétiques.

13. Composition selon la revendication 12, caractérisée par le fait que les huiles sont choisies parmi les huiles de silicone ou les isoparaffines.

14. Composition selon la revendication 13, caractérisée par le fait que les huiles de silicone sont des huiles à structure linéaire ou cyclique choisies parmi les polyalkylsiloxanes, les polyarylsiloxanes, les polyalkylarylsiloxanes et les polyorganosiloxanes modifiés par des groupements organofonctionnels différents du groupement thiol ou leurs mélanges.

15. Composition selon la revendication 11, caractérisée par le fait que les lotions huileuses contiennent en plus des cires, des résines ou gommes de silicone.

16. Composition selon la revendication 11, caractérisée par le fait qu'elle se présente sous forme d'émulsion non-ionique ou cationique.

17. Composition selon la revendication 16, caractérisée par le fait que la phase grasse des émulsions est constituée soit par le polydiorganosiloxane à fonction thiol défini dans l'une quelconque des revendications 7 à 10 soit par un mélange de ce polydiorganosiloxane à fonction thiol avec d'autres huiles ou cires.

18. Composition selon la revendication 16, caractérisée par le fait qu'elle se présente sous forme d'une émulsion non-ionique contenant un agent émulsionnant non-ionique, choisi parmi les alcools gras polyoxyéthylénés, les acides gras polyoxyéthylénés, les esters du sorbitan éventuellement polyoxyéthylénés, les alkylphénols polyoxyéthylénés ou polyglycérolés, les alcools gras et alpha-diols polyglycérolés.

19. Composition selon la revendication 11, caractérisée par le fait que la composition se présente sous forme d'une émulsion cationique contenant un agent émulsionnant cationique choisi parmi les halogénures d'ammonium quaternaire.

20. Composition selon la revendication 11, se présentant sous forme d'une dispersion aqueuse, caractérisée par le fait qu'elle contient en plus du polydiorganosiloxane à fonction thiol défini dans l'une quelconque des revendications 7 à 10, de l'eau et un agent de dispersion.

21. Composition selon la revendication 20, caractérisée par le fait que l'agent de dispersion est constitué par un copolymère d'acrylate d'ammonium et d'acrylamide réticulé.

22. Composition selon l'une quelconque des revendications 7 à 21, caractérisée par le fait qu'elle se présente sous forme de spray ou est pressurisée en aérosol.

23. Composition selon les revendications 7 à 10, caractérisé par le fait qu'elle se présente sous forme de lotion alcoolique, contenant outre le polydiorganosiloxane à fonction thiol, un alcool inférieur comportant 2 à 4 atomes de carbone et éventuellement d'autres solvants organiques.

## Claims

1. Use of a thiol-functional polydiorganosiloxane containing in its molecule at least one unit of formula: 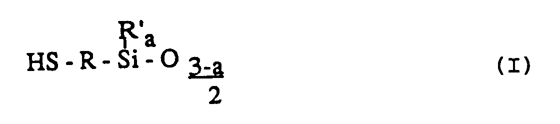 in which:
R denotes a divalent aliphatic radical having from 3 to 26 carbon atoms, optionally interrupted by an ester function and being able to carry ethylene oxide or propylene oxide units or mixtures thereof;
R' denotes a monovalent hydrocarbon radical having 1 to 6 carbon atoms, an alkoxy radical having 1 to 4 carbon atoms or a trimethylsilyloxy radical;
a denotes an integer equal to 0, 1 or 2, the remaining units having the formula: 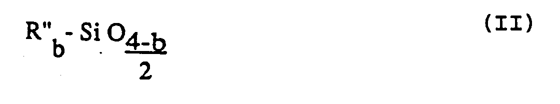 in which:
R'' represents a C₁-C₁₈ alkyl, phenyl(C₁-C₆)alkyl or phenyl group;
b is an integer denoting 1, 2 or 3; at least 50% of the groups R' and R'' representing a methyl radical, as an agent for protecting the colour of dyed keratinous fibres.

2. Use according to Claim 1, in which the polyorganosiloxane contains in addition units of formula: 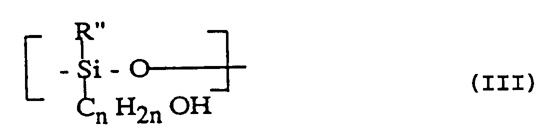 R'' has the meaning shown in Claim 1 and n is an integer from 1 to 18.

3. Use according to Claim 1 or 2, in which the polyorganosiloxane comprises a unit of formula (I) in which R represents an alkylene group having 3 to 8 carbon atoms, or a group of formula: -CₙH₂ₙO(CₓH₂ₓO)p-COCₘH₂ₘ-[sic] in which n denotes an integer of between 1 and 18, m denotes an integer of between 1 and 8, x = 2 or 3 and when x = 3 the C₃H₆ radical is branched, and p is equal to 0 or denotes a number that can go up to 40.

4. Use according to any one of Claims 1 to 3, characterised in that the thiol-functional polydiorganosiloxane corresponding to the definition shown in Claim 1 is chosen from the compounds in which R represents:
-(CH₂)₃-, 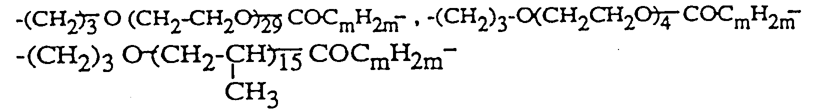 -(CH₂)₃O-COCH₂- 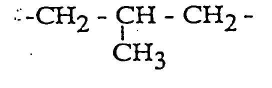 R' represents CH₃ or trimethylsilyloxy, R'' represents CH₃, C₁₂H₂₅ or 2-phenylpropyl, the total number of the (I) and (II) units being smaller than or equal to 500.

5. Use according to any one of Claims 1 to 4, characterised in that thiol-functional polydiorganosiloxanes are used in which the weight content of the thiol groups present is of between 0.1 and 15% and preferably of between 0.15 and 13%.

6. Use according to any one of Claims 1 to 5, characterised in that the thiol-functional polydiorganosiloxane is employed using a composition containing at least 0.1% by weight of the said thiol-functional polydiorganosiloxane in a support suitable for being applied to dyed keratinous fibres.

7. Composition intended to be applied to dyed keratinous fibres and in particular dyed human hair to protect them from the degradation due to external agents, characterised in that it contains in a medium suitable for application to keratinous fibres at least 0.1% of a thiol-functional polydiorganosiloxane containing in its molecule at least one unit of formula: 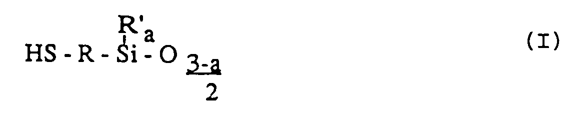 in which:
R denotes a group of formula:
-CₙH₂ₙO(CₓH₂ₓO)p-COCₘH₂ₘ-
in which n denotes an integer of between 1 and 18, m denotes an integer of between 1 and 8, x = 2 or 3 and when X = 3 the C₃H₆ radical is branched, and p is equal to 0 or denotes a number that can go up to 40;
R' denotes a monovalent hydrocarbon radical having 1 to 6 carbon atoms, an alkoxy radical having 1 to 4 carbon atoms or a trimethylsilyloxy radical;
a denotes an integer equal to 0, 1 or 2, the remaining units having the formula: 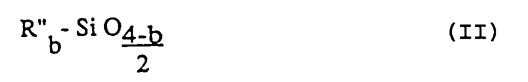 in which:
R'' represents a C₁-C₁₈ alkyl, phenyl(C₁-C₆)alkyl or phenyl group;
b is an integer denoting 1, 2 or 3; at least 50% of the groups R' and R'' representing a methyl radical.

8. Composition according to Claim 7, in which the polyorganosiloxane additionally contains units of formula: 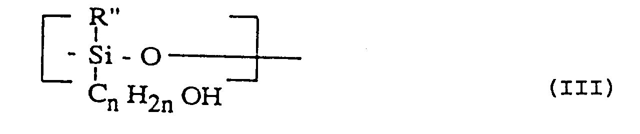 R'' has the meaning shown in Claim 7 and n is an integer from 1 to 18.

9. Composition according to Claim 7 or 8, characterised in that the thiol-functional polydiorganosiloxane is chosen from compounds in which R represents: 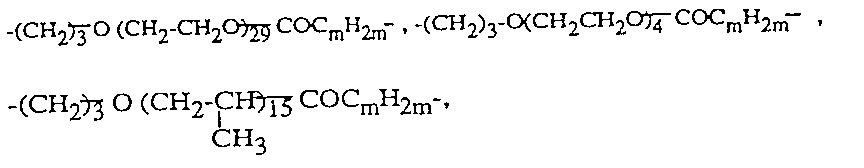 and -(CH₂)₃O-COCH₂-R' represents CH₃ or trimethylsilyloxy and R'' represents CH₃, C₁₂H₂₅ or 2-phenylpropyl, the total number of the (I) and (II) units being smaller than or equal to 500.

10. Composition according to any one of Claims 7 to 9, characterised in that use is made of thiol-functional polydiorganosiloxanes in which the weight content of thiol groups present is of between 0.1 and 15% and preferably between 0.15 and 13%.

11. Composition according to any one of Claims 7 to 10, characterised in that the composition is provided in the form of an oily lotion, emulsion or aqueous or aqueous/alcoholic dispersion.

12. Composition according to Claim 11, characterised in that the oily lotions contain, besides the thiol-functional polydiorganosiloxane, mineral, vegetable, animal or synthetic oils.

13. Composition according to Claim 12, characterised in that the oils are chosen from the silicone oils or the isoparaffins.

14. Composition according to Claim 13, characterised in that the silicone oils are oils of linear or cyclic structure chosen from the polyalkylsiloxanes, the polyarylsiloxanes, the polyalkylarylsiloxanes and the polyorganosiloxanes modified by organofunctional groups different from the thiol groups, or their mixtures.

15. Composition according to Claim 11, characterised in that the oily lotions contain in addition silicone gums, waxes or resins.

16. Composition according to Claim 11, characterised in that it is provided in the form of a nonionic or cationic emulsion.

17. Composition according to Claim 16, characterised in that the fatty phase of the emulsions consists either of the thiol-functional polydiorganosiloxane defined in any one of Claims 7 to 10 or of a mixture of this thiol-functional polydiorganosiloxane with other oils or waxes.

18. Composition according to Claim 16, characterised in that it is provided in the form of a nonionic emulsion containing a nonionic emulsifier chosen from the polyoxyethylenated fatty alcohols, the polyoxyethylenated fatty acids, the optionally polyoxyethylenated esters of sorbitan, the polyoxyethylenated or polyglycerolated alkylphenols or the polyglycerolated alpha-diols and fatty alcohols.

19. Composition according to Claim 11, characterised in that the composition is in the form of a cationic emulsion containing a cationic emulsifier chosen from the quaternary ammonium halides.

20. Composition according to Claim 11, being provided in the form of an aqueous dispersion, characterised in that it contains water and a dispersing agent in addition to the thiol-functional polydiorganosiloxane defined in any one of Claims 7 to 10.

21. Composition according to Claim 20, characterised in that the dispersing agent consists of a copolymer of crosslinked acrylamide and ammonium acrylate.

22. Composition according to any one of Claims 7 to 21, characterised in that it is provided in the form of a spray or is pressurised as an aerosol.

23. Composition according to Claims 7 to 10, characterised in that it is in the form of an alcoholic lotion containing, besides the thiol-functional polydiorganosiloxane, a lower alcohol containing 2 to 4 carbon atoms and, optionally, other organic solvents.

## Patentansprüche

1. Verwendung eines Polydiorganosiloxan mit Thiol-Funktion, enthaltend in seinem Molekül mindestens eine Einheit der Formel: 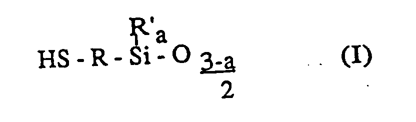 worin gilt:
R bedeutet einen 2-wertigen aliphatischen Rest mit 3 bis 26 Kohlenstoffatomen, der gegebenenfalls durch eine EsterFunktion unterbrochen ist und Ethylen- oder PropylenoxidEinheiten oder deren Mischung aufweisen kann,
R' bedeutet einen einwertigen Kohlenwasserstoffrest mit 1 bis 6 Kohlenstoffatomen, einen Alkoxy-Rest mit 1 bis 4 Kohlenstoffatomen oder eine Trimethylsilyloxy-Gruppe;
a bedeutet eine ganze Zahl von 0, 1 oder 2,
wobei die restlichen Einheiten die Formel aufweisen: 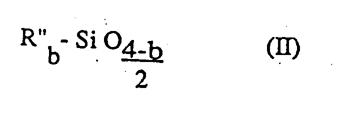 worin gilt:
R'' stellt eine C₁₋₁₈-Alkyl-, eine Phenyl-C₁₋₆-alkyl- oder eine Phenylgruppe dar;
b ist eine ganze Zahl von 1, 2 oder 3,
wobei mindestens 50% der Gruppen R' und R'' einen Methylrest darstellen,
als Mittel zum Schutz der Farbe gefärbter keratinischer Fasern.

2. Verwendung gemäß Anspruch 1, wobei das Polyorganosiloxan zusätzlich Einheiten der Formel enthält: 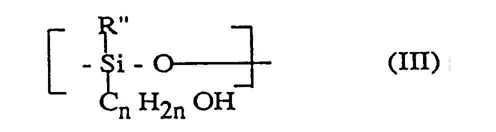 worin R'' die in Anspruch 1 angegebene Bedeutung hat und n eine ganze Zahl von 1 bis 8 ist.

3. Verwendung gemäß Anspruch 1 oder 2,
wobei das Polyorganosiloxan eine Einheit der Formel (I) enthält, worin R eine Alkylengruppe mit 3 bis 8 Kohlenstoffatomen oder eine Gruppe der Formel darstellt: 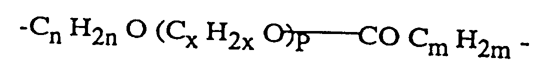 worin n eine ganze Zahl von 1 bis 18 und m eine ganze Zahl von 1 bis 8 bedeuten, x = 2 oder 3, und wenn x = 3, der Rest C₃H₆ verzweigt ist, und worin p = 0 ist oder eine Zahl bedeutet, die bis 40 gehen kann.

4. Verwendung gemäß einem der Ansprüche 1 bis 3,
dadurch **gekennzeichnet**, daß
das Polydiorganosiloxan mit Thiol-Funktion der in Anspruch 1 angegebenen Definition aus Verbindungen ausgewählt ist, in denen R darstellt:
-(CH₂)₃-, 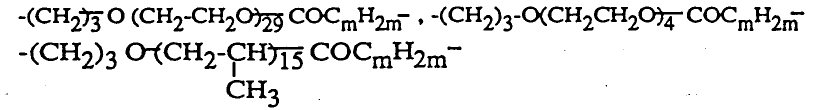 -(CH₂)₃O-COCH₂- 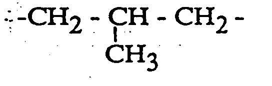 und in denen R' CH₃ oder eine Trimethylsilyloxy-Gruppe und R'' CH₃, C₁₂H₂₅ oder einen 2-Phenylpropylrest darstellen, wobei die Gesamtanzahl der Einheiten (I) und (II) weniger oder gleich 500 beträgt.

5. Verwendung gemäß einem der Ansprüche 1 bis 4,
dadurch **gekennzeichnet**, daß
man Polydiorganosiloxane mit Thiol-Funktion einsetzt, in denen der Gewichtsanteil der vorhandenen Thiol-Gruppen 0,1 bis 15 und vorzugweise 0,15 bis 13% beträgt.

6. Verwendung gemäß einem der Ansprüche 1 bis 5,
dadurch **gekennzeichnet**, daß
das Polydiorganosiloxan mit Thiol-Funktion verwendet wird, indem man eine Zusammensetzung zur Anwendung bringt, die mindestens 0,1 Gew.% des genannten Polydiorganosiloxan mit Thiol-Funktion in einer zur Aufbringung auf gefärbte keratinische Fasern geeigneten Trägerunterlage enthält.

7. Zusammensetzung zur Aufbringung auf gefärbte keratinische Fasern und insbesondere auf gefärbte menschliche Haare zu deren Schutz vor einem durch die Einwirkung äusserer Mittel hervorgerufenen Abbau,
dadurch **gekennzeichnet**, daß
sie in einem zur Aufbringung auf die keratinischen Fasern geeigneten Milieu mindestens 0,1% eines Polydiorganosiloxan mit Thiol-Funktion enthält, das in seinem Molekül mindestens eine Einheit der Formel aufweist: 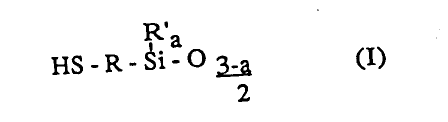 worin gilt:
R bedeutet eine Gruppierung der Formel: 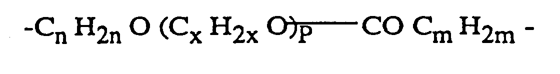 worin n eine ganze Zahl von 1 bis 18 und m eine ganze Zahl von 1 bis 8 bedeuten, x = 2 oder 3, und wenn x = 3, ist der Rest C₃H₆ verzweigt ist, und worin p gleich 0 ist oder eine ganze Zahl bedeutet, die bis 40 gehen kann;
R' bedeutet einen einwertigen Kohlenwasserstoffrest mit 1 bis 6 Kohlenstoffatomen, einen Alkoxy-Rest mit 1 bis 4 Kohlenstoffatomen oder eine Trimethylsilyloxy-Gruppe;
a bedeutet eine ganze Zahl von 0, 1 oder 2,
wobei die restlichen Einheiten die Formel aufweisen: 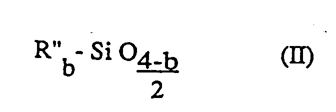 worin gilt:
R'' stellt eine C₁₋₁₈-Alkyl-, Phenyl-C₁₋₆-alkyl- oder eine Phenylgruppe dar;
b ist eine ganze Zahl von 1, 2 oder 3,
wobei mindestens 50% der Gruppen R' und R'' einen Methylrest darstellen.

8. Zusammensetzung gemäß Anspruch 7,
worin das Polyorganoxiloxan zusätzlich Einheiten der Formel aufweist: 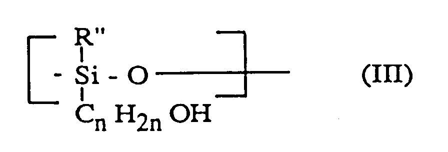 worin R'' die in Anspruch 7 angegebene Bedeutung hat und n eine ganze Zahl von 1 bis 18 ist.

9. Zusammensetzung gemäß Anspruch 7 oder 8,
dadurch **gekennzeichnet**, daß
das Polydioganosiloxan mit Thiol-Funktion aus Verbindungen ausgewählt ist, in denen R darstellt: 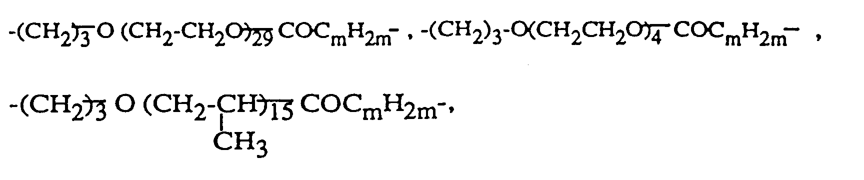 und
-(CH₂)₃O-COCH₂-
und in denen R' CH₃ oder eine Trimethylsilyloxy-Gruppe und R'' CH₃, C₁₂H₂₅ oder einen 2-Phenylpropylrest darstellen, wobei die Gesamtanzahl der Einheiten (I) und (II) weniger oder gleich 500 beträgt.

10. Zusammensetzung gemäß einem der Ansprüche 7 bis 9,
dadurch **gekennzeichnet**, daß
man Polydiorganosiloxane mit Thiol-Funktion zur Anwendung bringt, in denen der Gewichtsanteil der vorhandenen Thiolgruppe 0,1 bis 15 und vorzugsweise 0,15 bis 13% beträgt.

11. Zusammensetzung gemäß einem der Ansprüche 7 bis 10,
dadurch **gekennzeichnet**, daß
die Zusammensetzung in Form einer ölartigen Lotion, einer Emulsion, einer wässrigen oder hydroalkoholischen Dispersion vorliegt.

12. Zusammensetzung gemäß Anspruch 12,
dadurch **gekennzeichnet**, daß
die ölartigen Lotionen ausser dem Polydiorganosiloxan mit Thiol-Funktion mineralische, pflanzliche, tierische oder synthetische Öle enthalten.

13. Zusammensetzung gemäß Anspruch 12,
dadurch **gekennzeichnet**, daß
die Öle aus Silicon-Ölen oder Isoparaffinen ausgewählt sind.

14. Zusammensetzung gemäß Anspruch 13,
dadurch **gekennzeichnet**, daß
die Silicon-Öle Öle mit linearer oder zyklischer Struktur sind, die aus Polyalkylsiloxanen, Polyarylsiloxanen, Polyalkylarylsiloxanen und Polyorganosiloxanen, die mit organofunktionellen Gruppen modifiziert sind, die sich von der Thiolgruppe unterscheiden, oder aus deren Mischungen ausgewählt sind.

15. Zusammensetzung gemäß Anspruch 11,
dadurch **gekennzeichnet**, daß
die ölartigen Lotionen zusätzlich Wachse, Harze oder Gummiprodukte aus Silicon enthalten.

16. Zusammensetzung gemäß Anspruch 11,
dadurch **gekennzeichnet**, daß
sie in Form einer nicht-ionischen oder kationischen Emulsion vorliegt.

17. Zusammensetzung gemäß Anspruch 16,
dadurch **gekennzeichnet**, daß
die Fettphase der Emulsionen entweder aus dem in jedem der Ansprüche 7 bis 10 definierten Polydiorganosiloxan mit ThiolFunktion oder aus einer Mischung dieses Polydiorganosiloxan mit Thiol-Funktion mit weiteren Ölen oder Wachsen zusammengesetzt ist.

18. Zusammensetzung gemäß Anspruch 16,
dadurch **gekennzeichnet**, daß
sie in Form einer nicht-ionischen Emulsion vorliegt, die einen nicht-ionischen Emulgator enthält, der aus polyoxethylierten Fettalkoholen, polyoxethylierten Fettsäuren, gegebenenfalls polyoxethylierten Sorbitanestern, polyoxethylierten oder polyglycerierten Alkylphenolen, aus polyglycerierten Fettalkoholen und α-Diolen ausgewählt ist.

19. Zusammensetzung gemäß Anspruch 11,
dadurch **gekennzeichnet**, daß
die Zusammensetzung in Form einer kationischen Emulsion vorliegt, die einen kationischen Emulgator aus quaternären Anmoniumhalogeniden enthält.

20. Zusammensetzung gemäß Anspruch 11,
die in Form einer wässrigen Dispersion vorliegt,
dadurch **gekennzeichnet**, daß
sie zusätzlich zu dem in jedem der Ansprüche 7 bis 10 definierten Polydiorganosiloxan mit Thiol-Funktion Wasser und eine Dispergiermittel enthält.

21. Zusammensetzung gemäß Anspruch 20,
dadurch **gekennzeichnet,** daß
das Dispergiermittel aus einem vernetzten Copolymer aus Ammoniumacrylat und Acrylamid zusammengesetzt ist.

22. Zusammensetzung gemäß jedem der Ansprüche 7 bis 21,
dadurch **gekennzeichnet**, daß
sie in Form eines Spray oder eines unter Druck gesetzten Aerosol vorliegt.

23. Zusammensetzung gemäß einem der Ansprüche 7 bis 10,
dadurch **gekennzeichnet**, daß
sie in Form einer alkoholischen Lotion vorliegt, die ausser dem Polydiorganosiloxan mit Thiol-Funktion einen Niedrigalkohol mit 2 bis 4 Kohlenstoffatomen und gegebenenfalls weitere organische Lösungsmittel enthält.
